# EUROPEAN PATENT APPLICATION

(11) **EP 3 747 353 A1**
(43) Date of publication of application: **09.12.2020**
(21) Application number: 20175850.5
(22) Date of filing: 20.05.2020
(51) Int. Cl.: A61B 5/04, A61B 5/145, A61B 5/00, A61N 1/36

(54) **SYSTEM AND METHOD FOR PATIENT INTAKE MONITORING**

(30) Priority: 30.05.2019 US 201962854790 P; 25.03.2020 US 202016829985
(71) Applicant: Covidien LP, Mansfield, MA 02048 (US)
(72) Inventor: OSVALDO, Andres,Barrera, Madison, CT Connecticut 06443 (US); SARTOR, Joe, D, Longmont, CO Colorado 80504 (US)
(74) Representative: Maschio, Antonio

(57) **Abstract**

A system for monitoring patient intake includes an acquisition and transmission device having an electrode configured to detect vagus nerve activity. A first internal inductive coil is configured to communicate a signal indicative of the vagus nerve activity detected by the electrode to a second external inductive coil. The system also includes a processor configured to execute instructions stored in a memory that cause the system to process the signal received by the second induction coil into data corresponding to intake of the patient and communicate the data to a server that is accessible by a clinician.

## Description

### CROSS-REFERENCE TO RELATED APPLICATION

The present application claims the benefit of and priority to U.S. Provisional Patent Application Ser. No. 62/854,790, filed on May 30, 2019, the entire content of which is incorporated herein by reference.

### INTRODUCTION

Bariatric and diabetic patients, among others, need to monitor their intake of food and drink to improve compliance with lifestyle changes or to trigger events such as insulin delivery. Some existing intake and lifestyle monitoring systems for bariatric and diabetic patients rely on manual entries, which can be difficult to maintain.

### SUMMARY

In accordance with aspects of the disclosure, a system for monitoring patient intake includes an acquisition and transmission device configured to be implanted in a patient. The acquisition and transmission device includes an electrode configured to be coupled to a vagus nerve at a patient's stomach and to detect vagus nerve activity, and a first inductive coil operably coupled to the electrode. The electrode is configured to communicate a signal indicative of the detected vagus nerve activity to the first inductive coil. The system also includes a control unit configured to be positioned exterior to the patient and in proximity to the acquisition and transmission device. The control unit includes: a second inductive coil configured to receive from the first inductive coil the signal indicative of the detected vagus nerve activity; a processor; and a memory coupled to the processor. The memory stores instructions that, when executed by the processor, cause the control unit to process the signal received by the second inductive coil into data corresponding to intake of the patient, and communicate the data to a server for access by at least one of the patient or a clinician.

In an aspect of the disclosure, the electrode is configured to detect changes in a pH of the vagus nerve.

In another aspect of the disclosure, the server is a cloud-based server and the data communicated to the server is logged and configured to be accessed by at least one of the patient or the clinician.

In an aspect of the disclosure, the instructions, when executed by the processor, cause the control unit to display the data.

In another aspect of the disclosure, the control unit is coupled to a smartphone.

In yet another aspect of the disclosure, the first and second inductive coils are configured to communicate via near-field magnetic induction.

In an aspect of the disclosure, the acquisition and transmission device is configured to be implanted under the skin of the patient.

In another aspect of the disclosure, the control unit is configured to communicate with a computing device via at least one of WiFi, Bluetooth, BLE, or NFC.

In an aspect of the disclosure, the control unit is configured to communicate with at least one of an insulin pump, a continuous glucose monitor (CGM), or a monitor of ketonic bodies.

In yet another aspect of the disclosure, the instructions, when executed by the processor, cause the control unit to generate an alert based on a comparison between the data corresponding to intake of the patient and a pre-determined patient intake goal.

In yet another aspect of the disclosure, the alert is communicated to at least one of the patient or a clinician via at least one of a text message, a phone call, or an email.

In an aspect of the disclosure, the instructions, when executed by the processor, further cause the control unit to emit via the electrode a signal to stimulate the vagus nerve.

In accordance with aspects of the disclosure, a method for monitoring patient intake includes: detecting activity of a vagus nerve at a stomach of a patient via an electrode coupled to the vagus nerve; communicating a signal indicative of the detected vagus nerve activity from the electrode to a first inductive coil implanted within the patient; receiving, at a second inductive coil disposed exterior to the patient, the signal from the first inductive coil corresponding to the detected vagus nerve activity; processing the signal received from the first inductive coil into data corresponding to intake of the patient; and logging the data on the server for access by at least one of the patient or a clinician.

In an aspect of the disclosure, detecting vagus nerve activity includes detecting changes in a pH of the vagus nerve.

In another aspect of the disclosure, the method also includes displaying the data corresponding to intake of the patient via a device coupled to the second inductive coil.

In yet another aspect of the disclosure, the second inductive coil is disposed within a device coupled to a smartphone.

In an aspect of the disclosure, receiving the signal from the first inductive coil may include communicating the signal via near-field magnetic induction.

In another aspect of the disclosure, the method also includes comparing the data to a pre-determined patient intake goal and generating an alert to at least one of the patient or a clinician based on the comparison.

In accordance with aspects of the disclosure, an acquisition and transmission device for monitoring patient intake includes an electrode configured to be coupled to a vagus nerve at a patient's stomach and to detect vagus nerve activity, and a first inductive coil operably coupled to the electrode. The electrode is configured to communicate a signal indicative of the detected vagus nerve activity to the first inductive coil. The first inductive coil is configured to communicate the signal to an external device.

In accordance with aspects of the present disclosure, a system for amplifying vagus nerve activity includes an acquisition and transmission device configured to be implanted in a patient. The acquisition and transmission device includes a pH sensor configured to be coupled to a vagus nerve at a patient's stomach, a stimulating electrode, and a first inductive coil operably coupled to the electrode. The stimulating electrode is configured to be coupled to the vagus nerve. The pH sensor is configured to determine a pH of the vagus nerve. The pH sensor is configured to communicate a signal indicative of the determined vagus nerve pH to the first inductive coil. The system also includes a control unit configured to be positioned exterior to the patient and in proximity to the acquisition and transmission device. The control unit includes: a second inductive coil configured to receive from the first inductive coil the signal indicative of the detected vagus nerve pH, a processor, and a memory coupled to the processor. The memory stores instructions that, when executed by the processor, cause the control unit to process the received signal and cause the stimulating electrode to stimulate the vagus nerve.

In yet another aspect of the disclosure, the stimulating electrode may amplify the signal indicative of the determined vagus nerve pH.

In an aspect of the disclosure, the system may further include positioning the stimulating electrode between a cortex of the patient and the vagus nerve.

In another aspect of the disclosure, the stimulating electrode may be disposed on a sphincter muscle and stimulate contracture of the sphincter.

Further details and aspects of exemplary embodiments of the disclosure are described in more detail below with reference to the appended figures. Any of the above aspects and embodiments of the disclosure may be combined without departing from the scope of the disclosure.

### BRIEF DESCRIPTION OF THE DRAWINGS

Various exemplary embodiments of the disclosure are described herein below with reference to the drawings, wherein:
FIG. 1 is a diagram of the various devices and entities which may form part of or interact with a system for monitoring patient intake, in accordance with an embodiment of the disclosure;
FIG. 2 is a simplified box diagram of particular components of the system shown in FIG. 1, in accordance with an embodiment of the disclosure;
FIG. 3 is side view of an aspect of the system shown in FIG. 1, in accordance with an embodiment of the disclosure; and
FIG. 4 shows a flowchart of an exemplary method for intake monitoring in accordance with an embodiment of the disclosure.

### DETAILED DESCRIPTION

Systems and methods for patient intake monitoring will now be described in detail with reference to the drawings in which like reference numerals designate identical or corresponding elements in each of the several views. It is to be understood that the disclosed embodiments are merely exemplary of the disclosure and the described intake monitoring systems may be embodied in various forms. Well-known functions or constructions are not described in detail to avoid obscuring the disclosure in unnecessary detail. Specific structural and functional details disclosed herein are not to be interpreted as limiting, but merely as a basis for the claims and as a representative basis for teaching one skilled in the art to variously employ the disclosure in virtually any appropriately detailed structure.

In this description, the term "clinician" is used generally to refer to medical personnel including doctors, nurses, and support personnel.

The disclosed systems and methods for patient intake monitoring may be used by patients to individually monitor and manage their own health, and/or by patients in conjunction with treatment providers for long-term monitoring and treatment of medical conditions. In addition, the disclosed systems and methods for patient intake monitoring may analyze collected data and provide treatment providers with potential diagnoses and treatment options during treatment of a monitored patient. Generally, the system includes an acquisition and transmission device implanted within the patient and a control unit *(e.g.,* a smartphone) configured to communicate with the implanted acquisition and transmission device from outside the patient. The acquisition and transmission device may be implanted under the skin of the patient *(e.g.,* during gastric surgery) to enable positioning of the control unit as close as possible to the acquisition and transmission device for purposes of communication therebetween. With this purpose in mind, each of the control unit and the acquisition and control device may include one or more magnets to facilitate magnetic attraction therebetween, which may aid the user in positioning the control unit in general alignment with the acquisition and transmission device. The acquisition and transmission device includes a first, internal inductive coil operably coupled with one or more electrodes that attach to the subdiaphragmatic vagus nerve on the patient's stomach. The one or more electrodes are configured to detect vagus nerve activity that may be reflected, for example, by changes in the pH of the vagus nerve resulting from patient intake. The control unit includes a second, external inductive coil that, when positioned in suitable proximity to the internal inductive coil, enables inductive communication (e.g., via near-field magnetic induction communication) of a signal from the internal inductive coil to the external inductive coil. The control unit includes suitable software (e.g., smartphone app) that processes the signal received by the external inductive coil into meaningful data relating to the patient's intake and communicates this data over a suitable wireless network to a server (e.g., cloud-based server) where it is logged as patient-specific data and is accessible by medical professionals and/or the patient for monitoring the patient's intake. The patient-specific data may also be presented on a display of the control unit and/or a display of a suitable mobile device *(e.g.,* smartphone, wearable technology, *etc*.) coupled with the control unit.

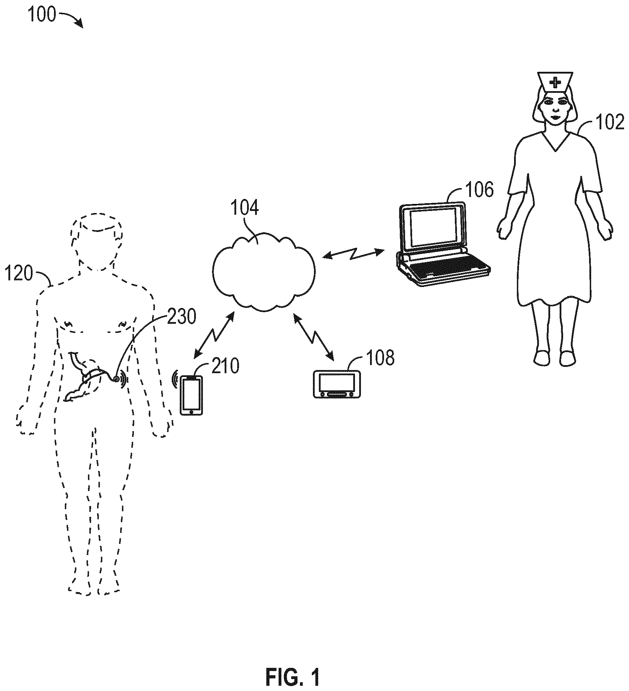

FIG. 1 shows a diagram of a system 100 for intake monitoring and data logging in accordance with an embodiment of the disclosure. The system 100 generally includes an acquisition and transmission device 230 implanted within a patient 120 and a control unit 210 disposed external to the patient 120 and configured to be positioned, by a clinician 102 or the patient 120, in suitable proximity to the implanted acquisition and transmission device 230 to facilitate communication therebetween. In some embodiments, the control unit 210 may be implemented as part of a mobile computing device *(e.g.,* a smart phone, or other mobile hand held device), a continuous glucose monitoring device (CGM), and/or an insulin pump, *etc.* The acquisition and transmission device 230 is configured to detect vagus nerve activity on the stomach of the patient 120 and inductively communicate a signal based on the detected vagus nerve activity to the control unit 210. The control unit 210 processes the communicated signal into data relating to the patient's intake and communicates this data over a suitable network to an off-site server 104 *(e.g.,* a cloud-based server) where it is logged as patient-specific data and is accessible by the clinician 102 via a suitable computing device 106. In some embodiments, the clinician 102 and/or patient 120 may also upload data to the server 104 via the suitable computing device 106. The system 100 may include additional servers 108 in communication with the server 104 to further process the patient-specific data and/or to make the patient-specific data accessible to additional groups or individuals such as, for example, research and development personnel, healthcare insurance providers, hospital personnel, *etc.* In some embodiments, data may also be sent to the server 104 and/or the server 108 for additional analysis such as detection of patterns and causes of success or failure for different patients and approaches to medical care. In various embodiments, the output of the control unit 210 may be combined with the output of at least one other sensor to improve accuracy and interpretation of the signal.

Depending on the laws and regulations of a particular jurisdiction in which the system 100 is to operate, one or more forms of consent and/or authorization may be required for patient data to be shared amongst the various entities described above. In such embodiments, the system 100 may be restricted to share patient-specific data with only the entities for which the patient 120 has consented and authorized data to be shared. Anonymized patient data and general analytics and statistics regarding patient data may also be shared among the various devices and entities of the system 100 where permitted.

Hospitals may be associated with the server 104 in a way that the hospital is capable of receiving patient health records from the server 104. Hospitals that are associated with the server 104 may be preferred by patients who have consented to having their patient health data collected by the server 104 because such hospitals may be better able to treat those patients for whom patient health records are maintained by the server 104. Additionally or alternatively, these hospitals may offer a variety of incentives and programs to the patients in exchange for making their health data available such as price reductions or streamlined appointment procedures and improved or enriched care engagement. In some instances, healthcare insurers may mandate that the patient make their data available to in-network hospitals as a requirement for coverage. This data regarding insured patients may be used to provide ever more accurate and even individualized pricing and risk assessment. Further, this data may be used to provide enticements and motivations to patients for modifying their behavior. For example, by reviewing activity levels, an insurance company can offer a price incentive to an individual who demonstrates, via the data, a commitment to decreased intake levels or other compliance with treatment plans. This type of change in behavior is beneficial to the patient from a health standpoint and beneficial to the insurance company in likely reduced risk of certain diseases, and thus, the enticement of a reduced premium for demonstrating this activity may lead to actual changes in behavior. A variety of other behavioral modifications and enticements for such modifications can be generated from the patient data and provided to the patient in an effort to improve the patient's health as well as reduce the costs and burdens on the medical systems associated with not addressing these behavioral issues.

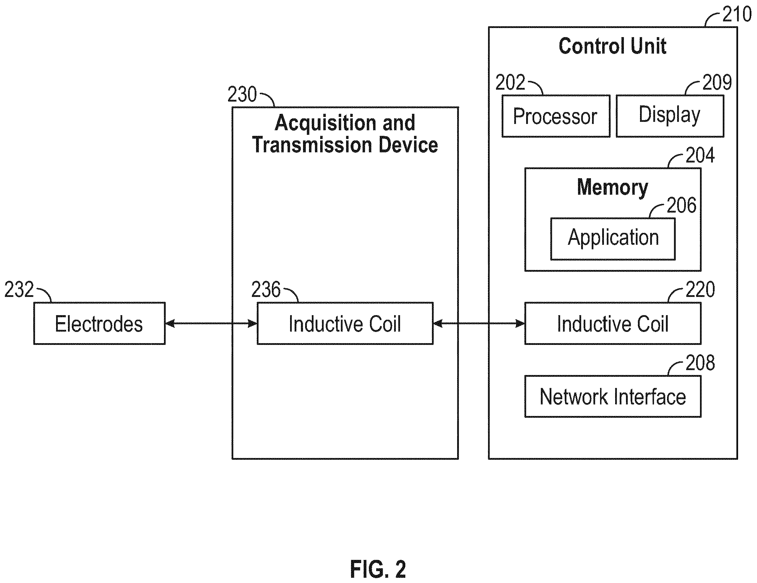

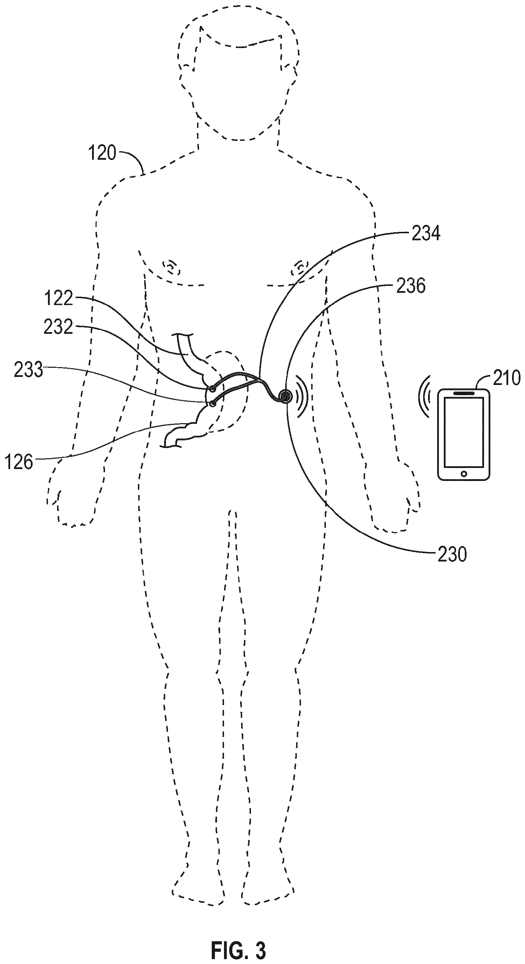

FIG. 2 shows the components of the acquisition and transmission device 230 and the control unit 210, which serve to enable operation of the acquisition and transmission device 230 and the control unit 210 within the system 100 of FIG. 1. The acquisition and transmission device 230 includes an inductive coil 236 that is operably coupled to one or more electrodes via suitable electrode leads 234 (FIG. 3). The one or more electrodes 232, such as, for example, sensors, are configured to be coupled to a vagus nerve 126 (FIG. 3) on the patient's stomach 122 to detect vagus nerve activity (e.g., changes in pH of the vagus nerve), which may be indicative of the patient's intake. As used herein, the term "coupled" to the vagus nerve means placed in contact with the vagus nerve, placed directly within the vagus nerve, or implanted in sufficiently close proximity to the vagus nerve to detect a physiological change (such as, for example, a change in pH) indicative of vagus nerve activity.

The control unit 210 includes a processor 202, a display 209, a memory 204, a software application 206 stored in memory 204 and executable by the processor 202, and a network interface 208 that facilitates data communication between the control unit 210 and the server 104 (FIG. 1) over a suitable wireless or wired network. The control unit 210 also includes an inductive coil 220 configured for communication with the inductive coil 236 of the acquisition and transmission device 230. The display 209 may be touch sensitive and/or voice activated, enabling the display 209 to serve as both an input and an output device. The control unit 210 may be any suitable electronic computing device on which a patient software application 206 may be installed and executed, examples of which include a personal computer (PC), smartphone, laptop, tablet, and/or a wearable computer such as a smart watch, *etc.*

The memory 204 includes any non-transitory computer-readable storage media for storing data and/or software that is executable by the processor 202 and which controls the operation of the control unit 210. In some embodiments, the memory 204 may include one or more solid-state storage devices such as flash memory chips. Alternatively, or in addition to the one or more solid-state storage devices, the memory 204 may include one or more mass storage devices connected to the processor 202 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 202. That is, computer readable storage media includes non-transitory, volatile and non-volatile, removable and non-removable media implemented in any method or technology for storage of information such as computer-readable instructions, data structures, program modules, or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by the control unit 210.

In some embodiments, the control unit 210 may connect via a wireless (e.g., Wi-Fi®, Bluetooth®, BLE®, and/or NFC®) or a wired connection to the server 104, the computing device 106, and/or a glucose monitoring system and insulin pump for constant glucose monitoring or for automatic dispensing of insulin. In some embodiments, the control unit 210 may connect to the server 104 and/or the computing device 106 for transmission and/or receiving of configuration parameters. In some embodiments, the control unit 210 may be hard wired to an insulin pump (not shown) to automatically administer insulin to the patient 120. For example, in order to administer insulin doses, close-loop insulin pumps may rely on reading of blood (or interstitial fluid) glucose concentration values. Nevertheless, there may be a physiological lag of a few minutes from the time of ingestion until the blood glucose concertation changes. In various embodiments, the output of the system may anticipate the blood glucose variations, and therefore may be used by the pump algorithms to anticipate or predict, for example, lows/highs and/or to determine insulin doses.

FIG. 3 shows the acquisition and transmission device 230 implanted within the patient 120 and the control unit 210 disposed external to the patient 120 and in suitable proximity to the inductive coil 236 to power the one or more electrodes 232 though the inductive coil and by resonance of the sensor side inductive coil 236 receive back a modulated signal indicative of the vagus nerve activity. The one or more electrodes 232 are coupled to the vagus nerve 126 of the stomach 122 for detecting vagus nerve activity such as changes in the pH of the vagus nerve 126. For bariatric patients, it may be necessary to remove at least a portion of a gastric sleeve (not shown) to couple the one or more electrodes 232 to the vagus nerve 126 on the stomach 122. Changes in pH (extracellular or within the vagus nerve) may be correlated with vagus nerve 126 activity. In some embodiments, the chemical pH signatures specific to vagus response to the gut hormone cholecystokinin (CCK) are measured by the electrodes 232. CCK is a gut hormone released during meal intake and is responsible for reducing appetite. CCK may be measured in-vitro with RIA (radioimmunoassay). In some embodiments, the one or more electrodes 232 may be Iridium Oxide (IrOx) electrodes suitable for sensing pH changes in the vagus nerve 126. See, *e.g.,* Simon C Cork et al., 2018 J. Neural Eng. 15 016001.

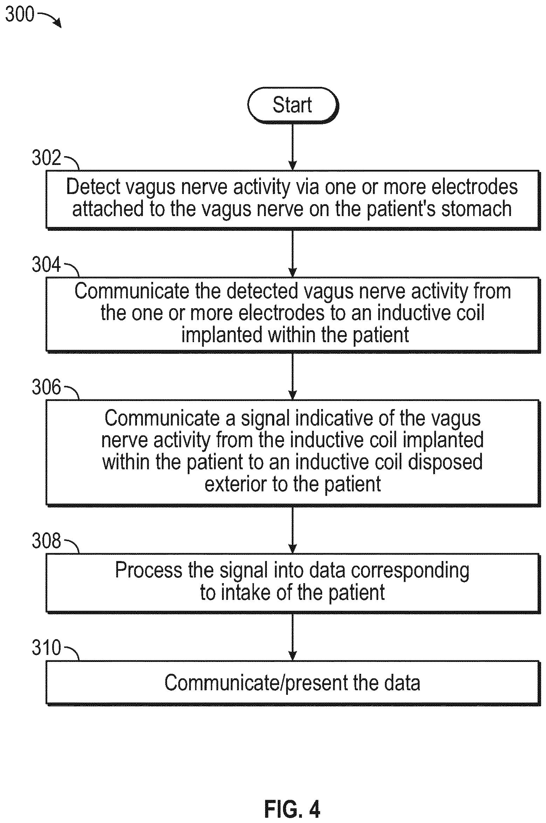

FIG. 4 shows a flowchart illustrating a method 300 for intake monitoring and data logging, in accordance with an embodiment of the disclosure. Those skilled in the art will appreciate that one or more steps of the method 300 may be performed in a different order, repeated, and/or omitted without departing from the scope of the disclosure.

Initially at step 302, one or more electrodes 232 coupled to the vagus nerve 126 of the patient's stomach 122 detect vagus nerve activity. The one or more electrodes 232 may be coupled to the vagus nerve 126 while the patient's stomach is accessible before, during, or after surgery. As described hereinabove, the one or more electrodes 232 may measure changes in the pH of the vagus nerve 126, or any other physiological change which may be indicative of patient intake. For example, accelerometers may be used to detect swallowing, or intra-stomach sensors may be used to detect food and fluids entering to the stomach. In various embodiments, other sensors may measure stomach and esophagus stretching and distension. At step 304, the vagus nerve activity detected by the one or more electrodes 232 is communicated to the inductive coil 236 from the one or more electrodes 232 via the electrode lead 234.

At step 306, the inductive coil 236 of the implanted acquisition and transmission device 230 inductively communicates a signal indicative of the detected vagus nerve activity to the inductive coil 220 of the control unit 210. In this manner, the inductive coil 236 may serve as a transmitter coil and the inductive coil 220 may serve as a receiver coil. As described hereinabove, the control unit 210 is placed in suitable proximity to the implanted acquisition and transmission device 230 to facilitate inductive communication between the two inductive coils 236 and 220. Placement of the control unit 210 relative to the implanted acquisition and transmission device 230 may be aided by magnetic attraction utilizing one or magnets (not shown) coupled to each of the control unit 210 and the acquisition and transmission device 230.

At step 308, the software application 206 stored in the memory 204 of the control unit 210 processes the signal received by the inductive coil 220. The signal may be processed to calculate a correlation between the vagus nerve activity with a patient's intake (e.g., intake of food or drink) and to generate corresponding meaningful patient-specific data such as long term patient intake data. For example, corresponding meaningful patient-specific data may include time between PH signals events can be used to determine patients' ingestion patterns. In various embodiments, PH signals may be synchronized and correlated with patient's Continuous Glucose Monitoring (CGM) data to infer more accurate prediction of glucose levels. In various embodiments, PH signals may be integrated to patient's pulse rate and variability and correlated with digestion processes of different type of meals. In some embodiments, patient intake may be tracked on an application that runs on a mobile device *(e.g.,* a smartphone).

At step 310, the patient specific-data generated by the software application 206 may be displayed on the display 209 of the control unit 210 and/or communicated to and logged on the server 104 for access by the appropriate clinicians via, *e.g.,* the computing device 106.

In some embodiments, a clinician may set goals for the patient to achieve. Monitoring pH indicates glucose levels based on a physiological lag time of ingestion until the blood glucose concertation changes. PH variations occur (and can be detected) before glucose absorption and presence in the bloodstream; therefore PH preempts glucose level variations in blood. In various embodiments, the output of the system may anticipate the blood glucose variations. In various embodiments, the output of the acquisition and transmission device 230 may anticipate the blood glucose variations, and therefore can be used by the pump algorithms to anticipate doses and/or predict lows/highs and notify the patient so she/he can react *(e.g.* exercise, ingest certain type of nutrients, *etc*.) to accommodate for a current trend and prevent the said low/high. In various embodiments, the acquisition and transmission device 230 may make recommendations to the patient based on the pH readings for that patient. For example, correlated to other signals (*e.g.* CGM, heart rate, *etc*.), the pH reading may be used to infer certain characteristics of the ingested foods. Data processed by the system 100 may be compared to the set goals by a suitable software application (e.g., application 206) and, when a patient fails to achieve the set goals, the system 100 provides an alert to the appropriate clinician (e.g., via the computing device 106) and/or to the patient (*e.g.,* via the control unit 210). The output may be used to recommend and monitor different ingestion patterns, *e.g*., smaller/larger meals, more/less number of meals per day, more/less time in between meals, *etc.* A clinician may set a goal for the patient to keep their glucose below a certain value. The system 100, by logging data corresponding to the patient's intake (e.g., via detection of changes in pH of the patient's vagus nerve), makes it possible to compare the logged data to the set goal and generate an alert if the patient fails to or is failing to meet the set goal. In some embodiments, alerts may be provided via text message, phone call, email, or the like. In some embodiments, the alert may be provided by sending a signal to the brain, *i.e.* the stimulation of the vagus nerve in response to the detection of a pH signal on the vagus nerve. In some embodiments, the system 100 makes it possible to calculate a correlation between the logged data and data from other monitoring devices such as, for example, electronic bath scales, activity trackers, and/or glucose monitors.

In various embodiments, the vagus nerve activity detected by the one or more electrodes 232 *(e.g.,* pH sensors) may be processed, (e.g., amplified) and the processed signal may be used to stimulate the same vagus nerve or another vagus verve. In various embodiments, the nerve stimulation feature may be provided by the system 100, or by a separate device with either separate or shared electrodes. A feature of the processed signal is an improved effect of the original signal while retaining the natural timing of the signal. For example, in a case where the vagus nerve is weak or damaged, the one or more electrodes 232 may detect the vagus nerve activity. This signal may be communicated to the inductive coil 236 from the one or more electrodes 232 via the electrode lead 234. Next, the inductive coil 236 of the implanted acquisition and transmission device 230 inductively communicates a signal indicative of the detected vagus nerve activity to the inductive coil 220 of the control unit 210, where the signal may be amplified. The amplified signal may then be inductively communicated by the inductive coil 220 of the control unit 210 to the inductive coil 236 of the implanted acquisition and transmission device 230 for stimulating the same vagus nerve or another vagus nerve. In various embodiments, the system 100 may include a stimulating electrode 233 configured to stimulate the vagus nerve 126 based on the amplified signal.

In various embodiments, the implanted acquisition and transmission device 230 may include a third inductive coil (not explicitly shown), and the control unit 210 may include a fourth inductive coil (not explicitly shown). The third and fourth inductive coils may be configured for providing near-field communication of a signal to the stimulating electrode 233. The third and fourth inductive coils may operate at a different frequency than the inductive coil 236 of the implanted acquisition and transmission device 230 and the inductive coil 220 of the control unit 210. The stimulating electrode 233 may be located between a cortex of the patient and the stomach end of the vagus nerve 126. In various embodiments, the amplification is used to stimulate a sphincter muscle in the lower esophagus or at the stomach pylori.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described embodiments. Accordingly, the disclosure is not to be limited by what has been particularly shown and described, except as indicated by the appended claims.

The invention maybe described by reference to the following numbered paragraphs:-
1. A system for monitoring patient intake, the system comprising:
   an acquisition and transmission device configured to be implanted in a patient, the acquisition and transmission device including:
   an electrode configured to be coupled to a vagus nerve at a patient's stomach and to detect vagus nerve activity; and
   a first internal inductive coil operably coupled to the electrode, the electrode configured to communicate a signal indicative of the detected vagus nerve activity to the first inductive coil; and
   a control unit configured to be positioned exterior to the patient and in proximity to the acquisition and transmission device, the control unit including:
      a second external inductive coil configured to receive from the first inductive coil the signal indicative of the detected vagus nerve activity;
      a processor; and
      a memory coupled to the processor, the memory having instructions stored thereon that, when executed by the processor, cause the control unit to:
         process the received signal into data corresponding to intake of the patient; and
         communicate the data to a server for access by at least one of the patient or a clinician.
2. The system according to paragraph 1, wherein the electrode is configured to detect changes in a pH of the vagus nerve.
3. The system according to paragraph 1, wherein the server is a cloud-based server and the data communicated to the server is logged to be accessed by at least one of the patient or the clinician.
4. The system according to paragraph 1, wherein the instructions, when executed by the processor, cause the control unit to display the data.
5. The system according to paragraph 1, wherein the first and second inductive coils are configured to communicate via near-field magnetic induction.
6. The system according to paragraph 1, wherein the acquisition and transmission device is configured to be implanted under skin of the patient.
7. The system of paragraph 1, wherein the control unit is configured to communicate with at least one of an insulin pump, a continuous glucose monitor (CGM), or a monitor of ketonic bodies.
8. The system according to paragraph 1, wherein the instructions, when executed by the processor, cause the control unit to generate an alert based on a comparison between the data corresponding to intake of the patient and a set patient goal.
9. The system of paragraph 8, wherein the alert is communicated to at least one of the patient or a clinician via at least one of a text message, a phone call, or an email.
10. The system of paragraph 8, wherein the control unit updates the goals in a case where the goals are achieved.
11. The system according to paragraph 1, wherein the instructions, when executed by the processor, further cause the control unit to emit, via the electrode, a signal to stimulate the vagus nerve.
12. A method for monitoring patient intake, the method comprising:
   detecting activity of a vagus nerve at a stomach of a patient via an electrode coupled to the vagus nerve;
   communicating a signal indicative of the detected vagus nerve activity from the electrode to a first internal inductive coil implanted within the patient;
   receiving, at a second external inductive coil disposed exterior to the patient, the signal from the first inductive coil indicative of the detected vagus nerve activity;
   processing the signal received from the first inductive coil into data corresponding to intake of the patient; and
   logging the data on a server for access by at least one of the patient or a clinician.
13. The method according to paragraph 12, wherein detecting vagus nerve activity includes detecting changes in a pH of the vagus nerve.
14. The method according to paragraph 13, further comprising displaying the data via a device coupled to the second inductive coil.
15. The method according to paragraph 12, wherein receiving the signal from the first inductive coil includes communicating the signal via near-field magnetic induction.
16. The method according to paragraph 12, further comprising:
   comparing the data to a set patient goal; and
   generating an alert to at least one of the patient or a clinician based on the comparison.
17. A system for amplifying vagus nerve activity, the system comprising:
   an acquisition and transmission device configured to be implanted in a patient, the acquisition and transmission device including:
   a pH sensor configured to be coupled to a vagus nerve at a patient's stomach and to determine a pH of the vagus nerve;
   a stimulating electrode configured to be coupled to the vagus nerve; and
   a first inductive coil operably coupled to the pH sensor, the pH sensor configured to communicate a signal indicative of the determined vagus nerve pH to the first inductive coil; and
   a control unit configured to be positioned exterior to the patient and in proximity to the acquisition and transmission device, the control unit including:
      a second inductive coil configured to receive from the first inductive coil the signal indicative of the detected vagus nerve pH;
      a processor; and
      a memory coupled to the processor, the memory having instructions stored thereon that, when executed by the processor, cause the control unit to:
         process the received signal; and
         cause the stimulating electrode to stimulate the vagus nerve.
18. The system according to paragraph 17, wherein the stimulating electrode amplifies the signal indicative of the determined vagus nerve pH.
19. The system according to paragraph 17, further comprising positioning the stimulating electrode between a cortex of the patient and the vagus nerve.
20. The system according to paragraph 17, further comprising stimulating contracture of a sphincter with the stimulating electrode.

## Claims

1. A system for monitoring patient intake, the system comprising:
an acquisition and transmission device configured to be implanted in a patient, the acquisition and transmission device including:
an electrode configured to be coupled to a vagus nerve at a patient's stomach and to detect vagus nerve activity; and
a first internal inductive coil operably coupled to the electrode, the electrode configured to communicate a signal indicative of the detected vagus nerve activity to the first inductive coil; and
a control unit configured to be positioned exterior to the patient and in proximity to the acquisition and transmission device, the control unit including:
a second external inductive coil configured to receive from the first inductive coil the signal indicative of the detected vagus nerve activity;
a processor; and
a memory coupled to the processor, the memory having instructions stored thereon that, when executed by the processor, cause the control unit to:
process the received signal into data corresponding to intake of the patient; and
communicate the data to a server for access by at least one of the patient or a clinician.

2. The system according to claim 1, wherein the electrode is configured to detect changes in a pH of the vagus nerve.

3. The system according to claim 1 or claim 2, wherein the server is a cloud-based server and the data communicated to the server is logged to be accessed by at least one of the patient or the clinician.

4. The system according to any preceding claim, wherein the instructions, when executed by the processor, cause the control unit to display the data.

5. The system according to any preceding claim, wherein the first and second inductive coils are configured to communicate via near-field magnetic induction; and/or wherein the acquisition and transmission device is configured to be implanted under skin of the patient.

6. The system of any preceding claim, wherein the control unit is configured to communicate with at least one of an insulin pump, a continuous glucose monitor (CGM), or a monitor of ketonic bodies; and/or wherein the instructions, when executed by the processor, cause the control unit to generate an alert based on a comparison between the data corresponding to intake of the patient and a set patient goal; preferably wherein the alert is communicated to at least one of the patient or a clinician via at least one of a text message, a phone call, or an email.

7. The system of claim 4, wherein the control unit updates the goals in a case where the goals are achieved.

8. The system according to any preceding claim, wherein the instructions, when executed by the processor, further cause the control unit to emit, via the electrode, a signal to stimulate the vagus nerve.

9. A method for monitoring patient intake, the method comprising:
detecting activity of a vagus nerve at a stomach of a patient via an electrode coupled to the vagus nerve;
communicating a signal indicative of the detected vagus nerve activity from the electrode to a first internal inductive coil implanted within the patient;
receiving, at a second external inductive coil disposed exterior to the patient, the signal from the first inductive coil indicative of the detected vagus nerve activity;
processing the signal received from the first inductive coil into data corresponding to intake of the patient; and
logging the data on a server for access by at least one of the patient or a clinician.

10. The method according to claim 9, wherein detecting vagus nerve activity includes detecting changes in a pH of the vagus nerve.

11. The method according to claim 9 or claim 10, further comprising displaying the data via a device coupled to the second inductive coil.

12. The method according to any of claims 9 to 11, wherein receiving the signal from the first inductive coil includes communicating the signal via near-field magnetic induction.

13. The method according to any of claims 9 to 12, further comprising:
comparing the data to a set patient goal; and
generating an alert to at least one of the patient or a clinician based on the comparison.

14. A system for amplifying vagus nerve activity, the system comprising:
an acquisition and transmission device configured to be implanted in a patient, the acquisition and transmission device including:
a pH sensor configured to be coupled to a vagus nerve at a patient's stomach and to determine a pH of the vagus nerve;
a stimulating electrode configured to be coupled to the vagus nerve; and
a first inductive coil operably coupled to the pH sensor, the pH sensor configured to communicate a signal indicative of the determined vagus nerve pH to the first inductive coil; and
a control unit configured to be positioned exterior to the patient and in proximity to the acquisition and transmission device, the control unit including:
a second inductive coil configured to receive from the first inductive coil the signal indicative of the detected vagus nerve pH;
a processor; and
a memory coupled to the processor, the memory having instructions stored thereon that, when executed by the processor, cause the control unit to:
process the received signal; and
cause the stimulating electrode to stimulate the vagus nerve.

15. The system according to claim 14, wherein the stimulating electrode amplifies the signal indicative of the determined vagus nerve pH; and/or further comprising positioning the stimulating electrode between a cortex of the patient and the vagus nerve; preferably further comprising stimulating contracture of a sphincter with the stimulating electrode.
